(19) **Europäisches Patentamt / European Patent Office / Office européen des brevets**

(11) **EP 4 632 060 A1**

(12) **EUROPEAN PATENT APPLICATION**
published in accordance with Art. 153(4) EPC

(43) Date of publication:
**15.10.2025  Bulletin 2025/42**

(21) Application number: **23900674.5**

(22) Date of filing: **06.12.2023**

(51) International Patent Classification (IPC):
**C12N 1/00** (2006.01)        **C12M 1/00** (2006.01)

(52) Cooperative Patent Classification (CPC):
**C12M 1/00; C12N 1/00**

(86) International application number:
**PCT/JP2023/043574**

(87) International publication number:
**WO 2024/122560 (13.06.2024 Gazette 2024/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **06.12.2022   JP 2022194671**

(71) Applicant: **KAO CORPORATION
Tokyo 103-8210 (JP)**

(72) Inventor: **HORA, Toshiki
Wakayama-shi, Wakayama 640-8580 (JP)**

(74) Representative: **Vossius & Partner
Patentanwälte Rechtsanwälte mbB
Siebertstrasse 3
81675 München (DE)**

(54) **FERMENTATIVE PRODUCTION PROCESS SUPPORT METHOD**

(57)    In a fermentative production process support method, one or more processors executes: a step of repeating a prediction cycle including a first prediction step of acquiring a plurality of types of culture state indexes by inputting culture information at a certain culture time point to a learned model and a second prediction step of acquiring culture information at a next culture time point; a step of acquiring control policy trend information that is a set of indexes to be optimized for each culture time point and in which at least one of the plurality of types of culture state indexes is individually specified as an index to be optimized for each culture time point; and a step of determining an optimum value of the specific culture condition correspondingly at each culture time point so that a culture state index specified as an index to be optimized at each culture time point in the control policy trend information among the plurality of types of culture state indexes acquired in the first prediction step is optimized.

**FIG.3**

**Description**

TECHNICAL FIELD

The present invention relates to a technique for supporting a fermentative production process of generating a target object in a culture vessel in which a microorganism is cultured.

BACKGROUND ART

Patent Document 1 below discloses a cell culture process search method capable of searching for optimum culture conditions without repeating experiments in cell culture and bioproduction.
This method includes a process condition generating step of generating a plurality of process conditions, a culture result predicting step of acquiring cell culture prediction results for the plurality of process conditions, and an optimization process condition acquisition step of finding an optimum process condition from the culture prediction results. The culture result predicting step includes a process condition acquisition step, an uptake constraint condition acquisition step, an optimization calculation step, and a concentration change calculation step, and the optimization calculation step calculates a metabolic flow rate (consumption rate) with a mathematical model (metabolic circuit model) related to cell metabolism on the basis of a culture medium composition (culture medium component concentration) and an uptake constraint condition.

CITATION LIST

Patent Document 1: WO 2021/166824 A

SUMMARY OF THE INVENTION

According to the present invention, there is provided a fermentative production process support method, the method being a method for supporting a fermentative production process of generating a target object in a culture vessel in which a microorganism is cultured, in which one or more processors capable of using a learned model that inputs culture information at least including a specific culture condition and outputs a plurality of types of culture state indexes executes: a culture behavior prediction step of repeating, for each culture time point within a predetermined culture time, a prediction cycle including a first prediction step of acquiring the plurality of types of culture state indexes by inputting the culture information at a certain culture time point to the learned model and a second prediction step of acquiring the culture information at a next culture time point; a trend acquisition step of acquiring control policy trend information that is a set of indexes to be optimized for each culture time point and in which at least one of the plurality of types of culture state indexes is individually specified as an index to be optimized for each culture time point; and an optimal condition determination step of determining an optimum value of the specific culture condition correspondingly at each culture time point so that a culture state index specified as an index to be optimized at each culture time point in the acquired control policy trend information among the plurality of types of culture state indexes acquired in the first prediction step is optimized.
In addition, according to the present invention, it is possible to provide a fermentative production process support apparatus including at least a memory and the one or more processors described above, the fermentative production process support apparatus capable of executing the fermentative production process support method described above.
Furthermore, it is also possible to provide a program for causing a computer to execute the fermentative production process support method described above, and it is also possible to provide a recording medium readable by a computer recording such a program. The recording medium includes a non-transitory tangible medium.

BRIEF DESCRIPTION OF THE DRAWINGS

[Fig. 1] is a schematic diagram illustrating an example of a culture system.
[Fig. 2] is a diagram conceptually illustrating a hardware configuration example of an information processing apparatus capable of executing a fermentative production process support method according to a first and a second embodiments.
[Fig. 3] is a flow chart for explaining the fermentative production process support method according to the first embodiment.
[Fig. 4] is a flow chart for explaining the fermentative production process support method according to the second embodiment.

[Fig. 5] is a diagram illustrating a data flow of a culture behavior prediction in Examples.

[Fig. 6] is a table indicative of results of a demonstration experiment.

DESCRIPTION OF EMBODIMENTS

In the above-described method described as a background, a plurality of process conditions is generated, a culture prediction result is acquired for each process condition, and an optimum process condition is found from each of the culture prediction results.

However, this method does not allow appropriate process conditions to be acquired throughout the fermentative production process. This is because the culture medium environment is changing over time, and the microorganisms sequentially change their metabolic balance in response to the external environment, whereas the above-described method seems to ignore the temporal elements of the process conditions.

Even if the above-described method is executed for each culture time point, the amount of calculation becomes enormous, which is unrealistic as an implementation method.

The present invention has been made in view of such a viewpoint, and provides a technique capable of acquiring appropriate culture conditions for each culture time point in a fermentative production process.

The present invention can provide a technique capable of acquiring appropriate culture conditions for each culture time point in the fermentative production process.

Hereinafter, examples of preferred embodiments of the present invention (hereinafter, referred to as the present embodiment) will be described. Note that the present embodiment described below is an example, and the present invention is not limited to the following configuration.

The present embodiment described below is a method for supporting a fermentative production process, and fermentative production process support methods according to two embodiments will be exemplified below. The fermentative production process support method according to the first embodiment is referred to as a first support method, and the fermentative production process support method according to the second embodiment is referred to as a second support method.

First, common matters in the first support method and the second support method will be described.

In the fermentative production process, a target object is generated in a culture vessel in which a microorganism is cultured.

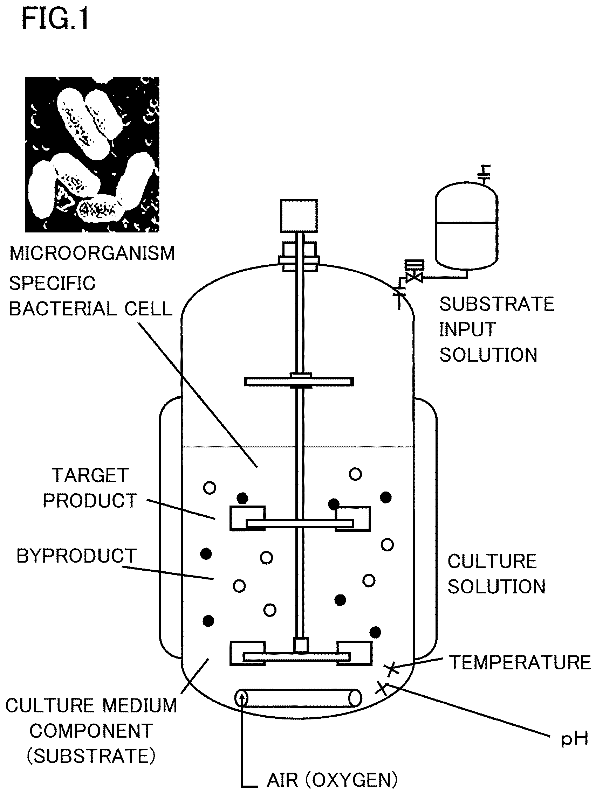

Fig. 1 is a schematic diagram illustrating an example of a culture system.

Fig. 1 exemplifies an example of a culture tank in which liquid culture is performed, and a microorganism is cultured in a culture solution in the culture tank (culture vessel).

In such a culture system, a temperature in the culture tank or the culture solution, a hydrogen ion index (pH) of the culture solution, and the like are measured, and charge of a substrate, which is a nutrient source necessary for the culture of microorganisms, supply of oxygen, and the like are controlled, so that production of a target object (target product) associated with the fermentative production process is promoted.

The first and second support methods support the optimization of the fermentative production process by acquiring appropriate culture conditions for each culture time point in such a fermentative production process.

The substrate is charged into the culture tank as a solution, and the solution is expressed as a substrate input solution in the present specification.

Oxygen is supplied by constantly flowing air from an air supply pipe at an arbitrary set flow rate where the air supply pipe is provided in a lower part of the culture tank and an exhaust pipe is provided in an upper part of the culture tank. Furthermore, an oxygen supply efficiency can be increased by stirring at an arbitrary set speed using a stirring blade.

In addition, in the culture behavior, as exemplified in Fig. 1, in addition to the target object, other substances (byproducts, carbon dioxide, and the like) are also generated.

However, the first and the second support methods are not intended only for the liquid culture exemplified in Fig. 1, but may be intended for solid culture.

In addition, it is sufficient that a type of microorganism and a culture medium can be determined according to a target object to be produced, and the present support method does not limit the microorganism to be cultured, the substrate, the target object, and the like.

Microorganisms include, for example, bacterial strains, strains belonging to fungal strains, undifferentiated cells and tissue cultures of animals or plants.

The culture medium can contain, for example, various components generally contained in the culture medium of microorganisms such as a carbon source, a nitrogen source, a metal salt such as a magnesium salt or a zinc salt, a sulfate, a phosphate, a pH adjusting agent, a surfactant, and an antifoaming agent.

Substrates include, for example, saccharides such as glucose, sucrose, and fructose, alcohols such as ethanol and methanol, organic acids such as citric acid, malic acid, and succinic acid, and waste molasses, and the like.

Target objects or byproducts include, for example, organic acids, amino acids, alcohols, antibodies, enzymes, coenzymes, vitamins, carbohydrates, fatty acids, and nucleic acids as substances generated by the metabolism of microorganisms.

[Hardware Configuration Example]

The first and the second support methods are executed by one or more processors included in one or more information processing apparatuses.

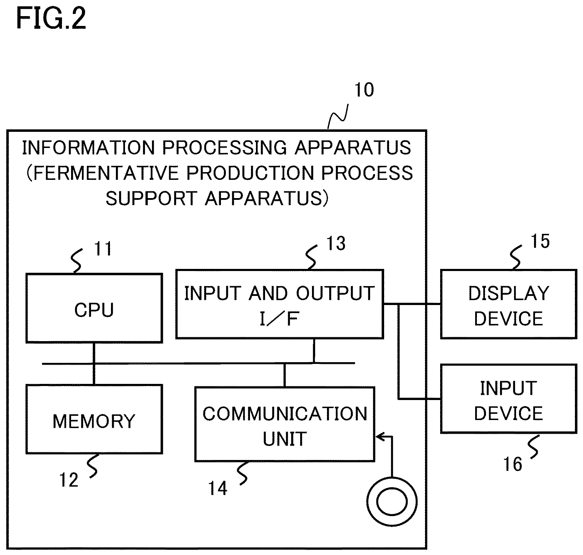

Fig. 2 is a diagram conceptually illustrating a hardware configuration example of an information processing apparatus 10 capable of executing the first and the second support methods.

The information processing apparatus 10 is a so-called computer, and includes a CPU 11, a memory 12, an input/output interface (I/F) 13, a communication unit 14, and the like. The information processing apparatus 10 may be a stationary personal computer (PC) or a portable terminal such as a portable PC, a smartphone, a tablet, and the like.

The CPU 11 is a so-called processor, and may include an application specific integrated circuit (ASIC), a digital signal processor (DSP), a graphics processing unit (GPU), and the like, in addition to a general central processing unit (CPU). The memory 12 is a random access memory (RAM), a read only memory (ROM), or an auxiliary storage device (such as a hard disk).

The input/output I/F13 can be connected to a user interface device such as a display device 15 and an input device 16. The display device 15 is a device that displays a screen corresponding to drawing data prepared by the CPU 11 or the like, such as a liquid crystal display (LCD) and a cathode ray tube (CRT). The input device 16 is a device that receives an input of a user operation such as a keyboard and a mouse. The display device 15 and the input device 16 may be integrated and realized as a touch panel.

The communication unit 14 performs communication with other computers via a communication network, exchange of signals with other devices such as a printer, and the like. A portable recording medium or the like can also be connected to the communication unit 14.

The hardware configuration of the information processing apparatus 10 is not limited to the example of Fig. 2. The information processing apparatus 10 may include other hardware elements not shown in the drawings. In addition, the number of hardware elements is not limited to the example of Fig. 2. For example, the information processing apparatus 10 may include a plurality of the CPUs 11. In addition, the information processing apparatus 10 may be realized by a plurality units of computers including a plurality of housings.

The information processing apparatus 10 can execute the first and the second support methods by the CPU 11 executing a computer program stored in the memory 12. In addition, it can also be expressed that the CPU 11 can execute the first and the second support methods through execution of the computer program stored in the memory 12. This computer program is installed from, for example, a portable recording medium such as a compact disc (CD) and a memory card, or another computer on a network via the input/output I/F13 or the communication unit 14, and then stored in the memory 12.

[Learned Model]

The information processing apparatus 10 (CPU 11) can use a learned model that outputs a plurality of types of culture state indexes by inputting culture information. The learned model can be obtained by machine learning in which teacher data including a plurality of combinations of the culture information and the plurality of types of culture state indexes is used.

Therefore, the learned model can also be referred to as a culture behavior prediction model.

In addition, the information processing apparatus 10 can also be referred to as a fermentative production process support apparatus capable of using a learned model and executing a fermentative production process support method.

The "culture information" is information related to the culture behavior of the microorganism, at least includes a specific culture condition, and is input to the learned model.

The culture information may include other information that is difficult to refer to as culture conditions. For example, as described later, the culture information may include a concentration, a change rate (generation rate, consumption rate, and the like), and a generation state of a specific component such as a target object, a byproduct, carbon dioxide, and a substrate in the culture vessel. In addition, the culture information may include information indicating bacterial cell activity state of the microorganism in the culture vessel. For example, the information may be information indicating whether the bacterial cell activity state of the microorganism in the culture vessel is in a growth phase of a bacterial cell or in a production phase of the target object by a categorical variable, or information indicating the bacterial cell growth rate.

The "specific culture condition" is a culture condition that can affect the fermentative production process, and is a culture condition for which an optimum value is to be obtained by the present support method. The specific culture condition is, for example, the temperature in the culture vessel, the hydrogen ion index (pH) of the culture solution in the culture vessel, an oxygen transfer rate, an oxygen consumption rate, and the like. In addition, the substrate concentration in the culture solution may also be included in the specific culture condition. However, the substrate concentration does not have to be treated as the specific culture condition, and may be treated as the concentration of the specific component in the culture vessel described above.

The "culture state index" is an index value capable of indicating the culture state in the culture vessel, and is information output from the learned model. The culture state index includes, for example, a concentration or a change rate of a specific component in the culture vessel, or both of them. The specific component herein may be any component present in the culture vessel, may be a component dissolved in the culture solution in the culture vessel, or may be a gas component in the culture vessel. In addition, the culture state index may include a bacterial cell state index such as a bacterial cell growth rate in the culture vessel.

The change rate of the specific component as the culture state index means a change rate of the substance amount of the specific component per unit bacterial cell mass (wet bacterial cell weight), and is, for example, some or all of the generation rate of the target object, the generation rate of the byproduct, the generation rate of carbon dioxide, or the consumption rate of the substrate, as described later.

As described above, the learned model in the present embodiment only needs to be constructed so as to be able to output a plurality of types of culture state indexes using culture information at least including a specific culture condition as an input, and concrete contents of the specific culture condition and the culture information and the concrete culture state indexes are not limited.

In addition, preferably, the learned model in the present embodiment is constructed so as to output only the plurality of types of culture state indexes using only the culture information as an input, and the culture information and the culture state indexes are configured by only an intensive variable or a dimensionless number, or both of them.

The temperature, the hydrogen ion index, the oxygen transfer rate ($mol/m^3/h$), and the oxygen consumption rate ($mol/m^3/h$) as the specific culture condition included in the culture information are also intensive variables or dimensionless numbers. In addition, the change rate of the specific component as the culture state index is indicated, for example, by a change rate of a substance amount (mol/g/h) per unit bacterial cell amount (per 1 g of bacterial cell), and can be said to be an intensive variable. Furthermore, the concentration of the characteristic component as the culture state index is also indicated by, for example, the component content weight (g/L) per unit volume of the culture solution, and can be said to be an intensive variable.

As described above, by setting input and output of an AI model to an intensive variable or a dimensionless number, culture behavior prediction independent of a shape or capacity of the culture vessel (culture tank) is possible. In other words, even a culture behavior in a culture tank having a different shape and capacity from the culture tank from which teacher data is obtained can be predicted with high accuracy.

Furthermore, the learned model in the present embodiment may be a regression equation obtained by regression analysis or a neural network model obtained by a principal component analysis, deep learning, or the like, and a data structure, a learning algorithm, and the like of the model are not limited.

For example, the learned model is realized by a combination of a computer program and a parameter, a combination of a plurality of functions and parameters, or the like. In a case where the learned model is constructed by a neural network and an input layer, an intermediate layer, and an output layer are regarded as a unit of one neural network, the learned model may refer to one neural network or may refer to a combination of a plurality of neural networks. In addition, the learned model may be configured by a combination of a plurality of multiple regression equations, or may be configured by one multiple regression equation.

A learned model used in the example described later is configured by a combination of a plurality of function models, each of which is a function of expressing a bacterial cell activity response by approximation, using the culture information as an input variable and the culture state index as an output variable. The plurality of function models is constructed using regression of supervised learning, and each function model uses at least a part of culture information as an input of the learned model as an input variable, and uses one type of the plurality of types of culture state indexes as an output of the learned model as an output variable.

The learned model may be stored in the memory 12 in the information processing apparatus 10, or may be stored in a memory of another computer accessible by the information processing apparatus 10 through communication.

Note that a concrete learning algorithm for constructing the learned model is not limited at all.

Hereinafter, the learned model used in the first and the second support methods is denoted as an AI model. In addition, in the following description, an execution subject of the first and the second support methods is described as the CPU 11.

[First Embodiment]

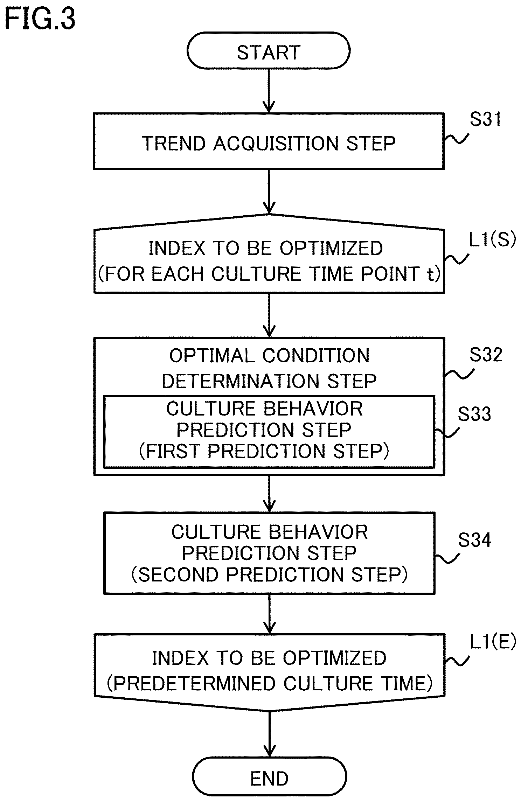

Hereinafter, details of the first support method will be described with reference to Fig. 3. Fig. 3 is a flow chart for explaining the fermentative production process support method according to the first embodiment.

The first support method mainly includes a trend acquisition step (S31), an optimal condition determination step (S32), and culture behavior prediction steps (S33) (S34).

Hereinafter, each of the steps will be described in detail.

In the trend acquisition step (S31), the CPU 11 acquires control policy trend information. Hereinafter, the control policy trend information may be abbreviated as a policy trend. The CPU 11 may read the policy trend stored in advance in the memory 12, may acquire the policy trend stored in a memory of another computer via communication, or may acquire the policy trend stored in a portable recording medium. In addition, the CPU 11 can display an input screen on the display device 15 and acquire the policy trend on the basis of data input by a user via the input screen.

The control policy trend information is a set of indexes to be optimized for each culture time point, and may be formed by one index to be optimized for each culture time point, may be formed by a plurality of indexes to be optimized for each culture time point, or may be formed by an arbitrary number of indexes to be optimized for each culture time point.

The index to be optimized is an index optimized in the determination of an optimum value in the optimal condition determination step (S32), in which at least one of the plurality of types of culture state indexes which is output information of the AI model is designated. In the present embodiment, an example will be described in which the policy trend is formed with one index to be optimized for each culture time point.

For example, in a case where the AI model is constructed so as to output the generation rate of the target object, the generation rate of the byproduct, the generation rate of carbon dioxide, and the consumption rate of the substrate as the culture state indexes, and where the generation rate of the target object, the generation rate of the byproduct, the generation rate of carbon dioxide, and the consumption rate of the substrate are indicated by identifiers "0", "1", "2", and "3", the policy trend is formed as follows.

```
Control policy trend information = [22223000011...]
```

When acquiring the policy trend, the CPU 11 reads the index to be optimized from the policy trend in the order of culture time points, and executes the optimal condition determination step (S32) and the culture behavior prediction steps (S33) (S34) on the basis of the index to be optimized for each culture time point (loop L1(S) to loop L1(E)).

The culture behavior prediction step is a step of predicting the culture behavior in the culture vessel by repeating, for each culture time point within a predetermined culture time, a prediction cycle including a first prediction step (S33) and a second prediction step (S34).

In the first prediction step (S33), the CPU 11 acquires the plurality of types of culture state indexes by inputting culture information at a certain culture time point to the AI model. Although not illustrated in Fig. 3, the culture information at the initial culture time point (0) includes the initial value of the specific culture condition and is acquired from the memory 12.

In the second prediction step (S34), the CPU 11 acquires culture information at the next culture time point. As described above, the second prediction step (S34) is a preparation step for the next prediction cycle.

There are various modes of acquisition methods of culture information in the second prediction step (S34). There is a mode in which some or all of the culture information at the next culture time point is acquired from a culture information list in which culture information determined in advance for each culture time point is stored. There may also be a mode in which some or all of the culture information at the next culture time point is calculated using some or all of the plurality of types of culture state indexes acquired in the immediately preceding first prediction step.

Here, a mode will be exemplified in which the AI model is constructed so that the culture information includes the concentration of the target object in addition to the specific culture condition and includes the generation rate of the target object as one of the plurality of types of culture state indexes. In this mode, in the first prediction step (S33), the CPU 11 acquires the plurality of types of culture state indexes including the generation rate of the target object by inputting the culture information including the specific culture condition and the concentration of the target object at the certain culture time point to the AI model. In the second prediction step (S34), the CPU 11 calculates the concentration of the target object at the next culture time point at least using the generation rate of the target object included in the plurality of types of culture state indexes acquired in the first prediction step (S33). For the calculation of the concentration of the target object, a calculation expression such as a substance balance equation as described later is used.

The specific culture condition includes, for example, a temperature in the culture vessel, a hydrogen ion index of the culture solution in the culture vessel, and an oxygen transfer rate or an oxygen consumption rate, and an optimum value of the above-mentioned specific culture condition is correspondingly determined for each culture time point. A candidate value for deriving the initial value and the optimum value of the specific culture condition can be acquired from the culture information list stored in the memory 12.

The oxygen consumption rate (OCR) means an oxygen consumption rate ($mol/m^3/h$) per unit volume of the culture solution by the microorganism in the culture vessel.

The oxygen transfer rate (OTR) means an oxygen transfer rate ($mol/m^3/h$) per unit volume of the culture solution at which oxygen supplied into the culture vessel moves into the culture solution to become dissolved oxygen.

Regarding the oxygen consumption rate and the oxygen transfer rate, it is sufficient that either one is used in the same manner in the first and the second support methods. This is because oxygen supply becomes a bottleneck during culture since the rate at which oxygen is dissolved in an aqueous solution (culture solution) is slow. For example, by measuring the oxygen concentration and the air flow rate on each of the supply side and the discharge side of air in the culture system, it is possible to derive, from the difference in the amount of passing oxygen (mol/h) per unit time at each point, how much oxygen has been supplied per unit culture solution amount ($m^3$), that is, the oxygen transfer rate OTR ($mol/m^3/h$). On the other hand, since the amount of oxygen that can be dissolved in the culture solution is very small compared to the oxygen transfer (consumption) rate per unit time, according to the following balance equation of oxygen in the culture solution, the oxygen transfer rate OTR and the oxygen consumption rate OCR can be treated equally by regarding the value related to the dissolved oxygen concentration as almost 0.

$$\text{OTR (mol/m}^3/h) = \text{OCR (mol/m}^3/h) + \text{(dissolved oxygen concentration (g/m}^3)/\text{oxygen substance amount (g/mol))}/\Delta t(h)$$

Note that although gaseous oxygen is also present in the culture solution, only the oxygen in the solution is available in the bacterial cells, and the rate at which the oxygen dissolves in the aqueous solution (culture solution) is low. Therefore, the oxygen of the gas in the solution is ignored in the above balance equation.

In addition, the temperature in the culture vessel may be the temperature of the culture solution in the culture vessel, the temperature of the air in the culture vessel, or both of them.

In the first prediction step (S33), the CPU 11 acquires the plurality of types of culture state indexes including the generation rate of the target object by inputting the culture information including the concentration of the target object, the temperature, and the hydrogen ion index, and the oxygen transfer rate or the oxygen consumption rate to the AI model. In the second prediction step (S34), the CPU 11 determines the optimum value of the specific culture condition (temperature, hydrogen ion index, and oxygen transfer rate or oxygen consumption rate) determined in the optimal condition determination step (S32) as the specific culture condition at the next culture time point, and, at least using it and the generation rate of the target object acquired in the first prediction step (S33), calculates the concentration of the target object at the next culture time point.

As in this mode, by adding the target object concentration of the culture solution in the culture vessel to the input information and adding the generation rate of the target object to the output information, an AI model capable of estimating the culture behavior with high accuracy can be constructed.

Furthermore, by including such the specific culture condition (in particular, the oxygen transfer rate or the oxygen consumption rate) in addition to the target object concentration in the culture information input to the AI model, the estimation accuracy of the AI model can be improved, and eventually, the prediction accuracy of the culture behavior can be improved.

However, the specific culture condition may further include other information different from the temperature, the pH, the oxygen transfer rate, and the oxygen consumption rate, and the culture information may further include the concentration of the specific component in the culture vessel such as the substrate concentration in addition to the target object concentration.

The AI model may be constructed such that the culture information further includes bacterial cell activity information indicating the bacterial cell activity state of the microorganism by a categorical variable.

In this case, in the first prediction step (S33), the CPU 11 acquires the plurality of types of culture state indexes including the generation rate of the target object by inputting the culture information including the bacterial cell activity information in addition to the target object concentration and the specific culture condition at the certain culture time point to the AI model.

The bacterial cell activity information indicates, for example, as a bacterial cell activity state of the microorganism in the culture vessel, whether the state is in a growth phase of bacterial cell or in a production phase of the target object with a categorical variable (0 or 1).

By adding the bacterial cell activity information to the culture information input to the AI model in this manner, the bacterial cell activity response can be simulated in the AI model, and eventually the prediction accuracy of the culture behavior can be further improved.

In a case where the culture information includes bacterial cell activity information, the specific culture condition included in the culture information preferably includes an oxygen transfer rate or an oxygen consumption rate. This is because the estimation accuracy of the generation rate of the target object in the AI model can be enhanced by using the oxygen transfer rate or the oxygen consumption rate as the input information. Furthermore, since the bacterial cell activity state can be estimated using the oxygen transfer rate or the oxygen consumption rate, the oxygen transfer rate or the oxygen consumption rate can be used for calculating bacterial cell activity information at the next culture time point.

The AI model may be constructed such that the culture information further includes byproduct state information indicating an increase and decrease state of the byproduct in the culture vessel by a categorical variable.

In this case, in the first prediction step (S33), the CPU 11 acquires a generation rate of the target object and a generation rate of the byproduct by inputting culture information including the byproduct state information in addition to the target object concentration and the specific culture condition at the certain culture time point to the AI model.

The byproduct state information is information indicating an increase and decrease state of the byproduct accompanying the generation of the byproduct by the microorganism, and is, for example, information indicating whether the state is an increase state or a decrease state with a categorical variable (0 or 1). At this time, the byproduct is a substance corresponding to an intermediate in a metabolic pathway in which bacterial cells produce the target object. Therefore, the increased state of byproducts indicates a metabolic state in which the metabolism after the intermediate is stagnant in the metabolic pathway, and the decreased state of byproducts indicates a metabolic state in which the metabolism after the intermediate is promoted. In addition, the generation rate of the byproduct indicates a positive value in a metabolic state in which the metabolism after the intermediate is stagnant, and indicates a negative value in a metabolic state in which the metabolism after the intermediate is promoted. Note that the specific component in this case indicates the target object or the substrate.

By adding the byproduct state information to the culture information input to the AI model in this manner, the bacterial cell activity response can be simulated in the AI model, and eventually the prediction accuracy of the culture behavior can be further improved.

In the mode of calculating the concentration of the target object at the next culture time point using the generation rate of the target object, for example, the following substance balance equation can be used.

In the example of the following balance equation (Equation 1), $n_{TGT}$ represents the substance amount (mol) of the target object, $r_{TGT}$ represents the generation rate (mol/g/h) of the target object, and B(t) represents the bacterial cell mass (g) of the specific bacterial cell of the microorganism at the culture time point t. In addition, in the example of (Equation 2), $MW_{TGT}$ represents a molecular weight (g/mol) of the target object, V represents the culture solution amount (L) at the culture time point (t+1), and $C_{TGT}$ represents the target object concentration (g/L) in the culture solution at the culture time point (t+1).

In this case, in the second prediction step (S34), the CPU 11 can calculate the substance amount $n_{TGT}$ of the target object at the next culture time point (t+1) by solving the differential equation of (Equation 1), and can calculate the target object concentration $C_{TGT}$ in the culture solution at the next culture time point (t+1) by substituting the substance amount into (Equation 2).

[Mathematical Equation 1]

$$\frac{dn_{TGT}}{dt} = r_{TGT} \cdot B(t) \quad \cdots \text{(Equation 1)}$$

$$C_{TGT} = \frac{n_{TGT} \cdot MW_{TGT}}{V} \quad \cdots \text{(Equation 2)}$$

However, the substance balance equation used in the second prediction step (S34) is not limited to such an example.

B(t) and V in the above-describe (Equation 1) and (Equation 2) may be extracted from a value list determined in advance for each culture time point, or may be calculated by a substance balance equation or the like.

For example, as exemplified in the following (Equation 3), the CPU 11 can calculate the specific bacterial cell mass of microorganism at each culture time point on the basis of the culture elapsed time and the oxygen transfer rate or the oxygen consumption rate at each culture time point.

The specific bacterial cell mass calculated here means a wet weight or a dry weight of a specific type of bacterial cell of a microorganism cultured in a culture vessel, and may target at one type of bacterial cell or two or more types of bacterial cells.

In (Equation 3), min{} is a function that takes a smaller value between a value on the left side and a value on the right side separated by a comma. The left side shows a state in which the dissolved oxygen in the culture solution is excessive and the specific bacterial cells grow according to a time t, and the right side shows an upper limit state in which the dissolved oxygen is insufficient, the upper limit state being in accordance with the oxygen transfer rate or the oxygen consumption rate.

In addition, in (Equation 3), BCI represents an initial bacterial cell concentration of a specific bacterial cell of a microorganism in the culture vessel, and k1, k2, and k3 each are a fixed value. A value of the BCI is acquired as an initial value in the first step (S31), for example. k1, k2, and k3 are values obtained by experiments by the present inventors, and are, for example, k1 = 0.18, k2 = 17.5, and k3 = 0.334. However, k1, k2, and k3 may be appropriately changed according to the value of BCI, the type of microorganism, or the culture medium.

In (Equation 3), the oxygen transfer rate OTR may be replaced with the oxygen consumption rate OCR.

[Mathematical Equation 2]

$$\frac{B(t)}{V} = min\{BCI \cdot exp(k1 \cdot t),\ k2 \cdot OTR^{k3}\}$$

$$\cdots (\text{Equation 3})$$

In the example of (Equation 3) described above, a culture solution amount V at the next culture time point (t+1) may be extracted from a value list determined in advance or may be calculated by a substance balance equation or the like.

The CPU 11 can calculate the concentration of the target object at the next culture time point at least using the thus calculated specific bacterial cell mass and the generation rate of the target object acquired in the first prediction step (S33). When (Equation 1), (Equation 2), and (Equation 3) described above are used, the bacterial cell mass B(t) of the specific bacterial cell of the microorganism at the culture time point t is calculated by (Equation 3), the substance amount $n_{TGT}$ of the target object at the next culture time point (t+1) can be calculated by solving the differential equation of (Equation 1) using the calculated B(t) and the generation rate $r_{TGT}$ of the target object acquired in the first prediction step (S33), and the target object concentration at the next culture time point (t+1) can be calculated using (Equation 2).

In a case where the culture information input to the AI model includes bacterial cell activity information, in the second prediction step (S34), the CPU 11 acquires bacterial cell activity information at the next culture time point.

The next bacterial cell activity information may be acquired from a value list determined in advance for each culture time point, or may be calculated using an oxygen transfer rate or an oxygen consumption rate included in the specific culture condition.

In the latter case, for example, the bacterial cell activity information is set to a value (0) indicating that the bacterial cell activity state is in the growth phase of bacterial cell when the left side is selected (decreases) in the min{} function of (Equation 3) described above, and is set to a value (1) indicating that it is in the production phase of the target object when the right side is selected (decreases).

In addition, in a case where the culture information input to the AI model includes the byproduct state information, and the generation rate of the byproduct is acquired from the AI model as one of the change rates of the specific component, in the second prediction step (S34), the CPU 11 can acquire byproduct state information at the next culture time point using the acquired generation rate of the byproduct.

For example, the byproduct state information is set to a value (0) indicating the increasing state of the byproduct in a state where the generation rate of the byproduct is 0 or more, and is set to a value (1) indicating the decreasing state of the byproduct in a case where the generation rate of the byproduct is less than 0.

In addition, in a case where the AI model is constructed so that the culture state index, which is the output information, includes the generation rate of the target object and the substrate consumption rate, the substrate concentration in the culture solution can be calculated using the following substance balance equation or the like.

In the example of the following balance equation (Equation 4), $n_{SBS}$ represents the substance amount (mol) of the substrate, $r_{SBS}$ represents the consumption rate (mol/g/h) of the substrate, and B(t) represents the bacterial cell mass (g) of the specific bacterial cell of the microorganism at the culture time point t, $C_{Feed}$ represents the substrate concentration (g/L) in the substrate input solution, $F_{SBS}(t)$ represents the substrate input speed (L/h) at the culture time point t, and $MW_{SBS}$ represents the molecular weight (g/mol) of the substrate. In addition, in the example of (Equation 5), V represents the culture solution amount (L) at the culture time point (t+1).

In this case, the CPU 11 can acquire $C_{Feed}$ and $F_{SBS}(t)$ as substrate input information at the culture time point t, can calculate a substance amount $n_{SBS}$ of the substrate at the next culture time point (t+1) by solving the differential equation of Equation (4) using these pieces of substrate input information and the consumption rate $r_{SBS}$ of the substrate acquired from the AI model in the first prediction step (S33), and can calculate a substrate concentration $C_{SBS}$ in the culture solution at the next culture time point (t+1) by substituting the substance amount into (Equation 5). The acquisition method for the bacterial cell mass B(t) at the culture time point t is as described above.

[Mathematical Equation 3]

$$\frac{dn_{SBS}}{dt} = -r_{SBS} \cdot B(t) + \frac{C_{Feed}}{MW_{SBS}} \cdot F_{SBS}(t)$$

$$\cdots (\text{Equation 4})$$

$$C_{SBS} = \frac{n_{SBS} \cdot MW_{SBS}}{V} \qquad \cdots (\text{Equation 5})$$

However, the calculation equation for the substrate concentration is not limited to such an example. In addition, the substrate input information including $C_{Feed}$ and $F_{SBS}(t)$ is stored in the substrate information list for each culture time point, and the CPU 11 can acquire $C_{Feed}$ and $F_{SBS}(t)$ from the list as the substrate input information at the culture time point t.

In the optimal condition determination step (S32), the CPU 11 determines an optimum value of the specific culture condition correspondingly at each culture time point so that the culture state index specified, as the indexes to be optimized at each culture time point, in the policy trend acquired in the step (S21) is optimized, the culture state index being specified among the plurality of types of culture state indexes acquired from the AI model in the first prediction step (S33).

For example, in a case where the AI model is constructed so as to output the generation rate of the target object, the generation rate of the byproduct, the generation rate of carbon dioxide, and the consumption rate of the substrate as the culture state indexes, and where the identifier "2" of the generation rate of carbon dioxide is specified as the index to be optimized at the culture time point (t=2) in the policy trend, the value of the specific culture condition such that the generation rate of carbon dioxide is optimized among the four types of culture state indexes acquired from the AI model by inputting the culture information at the culture time point (t=2) (such that the generation rate becomes the fastest, the slowest, or a desired rate determined in advance) is determined as the optimum value.

In the optimal condition determination step (S32), various types of optimization methods can be used. For example, by using Bayesian optimization, calculation costs can be reduced.

The CPU 11 reads the indexes to be optimized in the order of the culture time points from the policy trend and executes each of the optimal condition determination step (S32) and the culture behavior prediction steps (S33) (S34) on the basis of the indexes to be optimized for each culture time point, thereby enabling to acquire the optimum value of the specific culture condition at each culture time point within the predetermined culture time.

That is, according to the first support method, it is possible to acquire an appropriate condition along the policy trend as the culture condition for each culture time point in the fermentative production process. Furthermore, by using a plurality of types of culture state indexes output from the AI model on the basis of the optimum value of the specific culture condition at each culture time point, culture result information such as the production amounts of the target object, the byproduct, and the like and the consumption amount of the substrate can also be acquired.

[Second Embodiment]

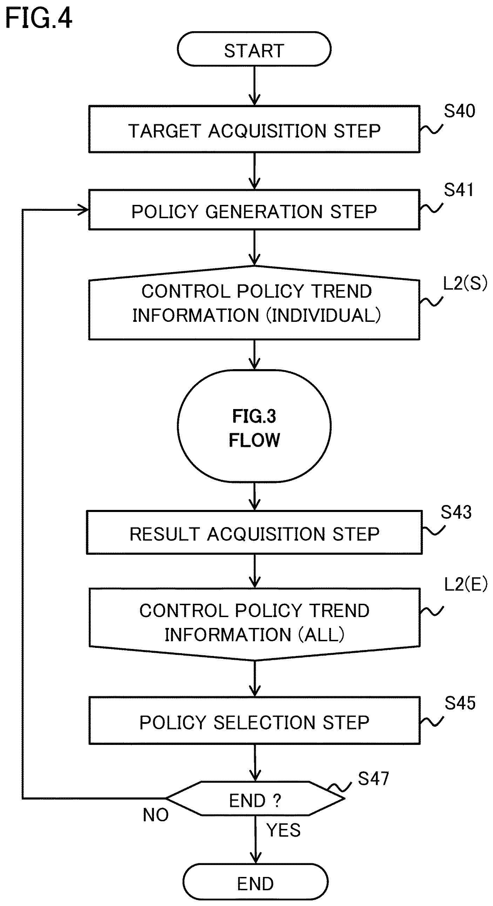

Next, details of the second support method will be described with reference to Fig. 4. Fig. 4 is a flow chart for explaining the fermentative production process support method according to the second embodiment.

The second support method includes all the steps constituting the first support method described above, and in the following description, contents related to the first support method will not be repeated. In Fig. 4, the steps of the first support method illustrated in Fig. 3 are included in a portion denoted as "FIG. 3 FLOW".

The second support method includes a target acquisition step (S40), a policy generation step (S41), a result acquisition step (S43), and a policy selection step (S45) in addition to the steps of the first support method.

Hereinafter, each of the steps will be described in detail.

In the target acquisition step (S40), the CPU 11 acquires target quality information.

The "target quality information" is information indicating the final target quality in the fermentative production process, and for example, maximization of a yield of the target object per input substrate, maximization of the production amount of the target object, minimization of a residual amount of a byproduct, minimization of a substrate residual amount, and the like are set.

The CPU 11 may read target quality information stored in advance in the memory 12, may acquire target quality information stored in a memory of another computer via communication, or may acquire target quality information stored in a portable recording medium. In addition, the CPU 11 can display an input screen on the display device 15 and acquire the target quality information on the basis of data input by a user via the input screen.

In the policy generation step (S41), the CPU 11 generates the plurality of pieces of control policy trend information. The control policy trend information is identical to that used in the first support method, and is also abbreviated here as the policy trend.

The CPU 11 generates a plurality of policy trends such that the indexes to be optimized at at least one culture time point are different from each other.

For example, the CPU 11 generates a plurality of policy trends using a genetic algorithm on the basis of one or more policy trends selected in a policy selection step (S45) to be described later. Specifically, since one or more excellent policy trends conforming to the target quality information are selected in the policy selection step (S45), the plurality of policy trends is generated by crossing (recombining) the indexes to be optimized for each culture time point indicated by the selected excellent policy trend or by mutating (changing) a predetermined ratio (several %) of the indexes to be optimized.

By using the genetic algorithm for generating the policy trends in this manner, optimization of the policy trend can be efficiently performed, and eventually, calculation costs can be reduced.

However, the optimization algorithm used for the generation method of the policy trends in the step (S41) is not limited.

After the execution of the policy generation step (S41), the CPU 11 selects the policy trends one by one, and executes the steps of the first support method and the result acquisition step (S43) on the basis of each of the policy trends (loop L2(S) to loop L2(E)).

When the first support method is executed correspondingly on the basis of each policy trend, the optimum value of the specific culture condition at each culture time point within the predetermined culture time is correspondingly acquired along each policy trend, and the culture behavior prediction step at each culture time point is executed correspondingly using the optimum value of the specific culture condition.

Consequently, in the result acquisition step (S43), the CPU 11 can acquire a culture result index over a predetermined culture time for each policy trend as a result of the culture behavior prediction (execution of the first prediction step (S33) and the second prediction step (S34)) at each culture time point using the specific culture condition optimized in accordance with each policy trend.

As a result of the culture behavior prediction at each culture time point within a predetermined culture time, information such as a production amount of the specific component (target object, byproduct, carbon dioxide, and the like), an input amount, a consumption amount and a residual amount of the substrate in the culture vessel over the predetermined culture time can be acquired.

The CPU 11 acquires a culture result index conforming to the target quality information by processing (calculating) such information so as to conform to the quality indicated by the target quality information acquired in the step (S40). For example, in a case where the target quality information indicates the maximization of a yield of a target object per input substrate, the CPU 11 acquires the yield of the target object as the culture result index by dividing the production amount of the target object by the substrate input amount. In addition, in a case where the target quality information indicates the maximization of the production amount of the target object, the production amount of the target object is acquired as the culture result index, in a case where the target quality information indicates the minimization of the residual amount of the byproduct, the production amount of the byproduct is acquired as the culture result index, and in a case where the target quality information indicates the minimization of the substrate residual amount, the substrate residual amount is acquired as the culture result index.

When the steps of the first support method and the result acquisition step (S43) are executed for all the policy trends generated in the step (S41), the loop L2 is exited and the policy selection step (S45) is executed.

In the policy selection step (S45), the CPU 11 selects one or more policy trends conforming to the target quality information from among the plurality of policy trends on the basis of the culture result index acquired for each policy trend. Here, one policy trend corresponding to the culture result index having the highest degree of conformity to the target quality indicated by the target quality information may be selected, or a plurality of policy trends may be selected in descending order of the degree of conformity to the target quality of the culture result index. In a case where the genetic algorithm is used in the step (S41), for example, two policy trends are selected in descending order of the degree of conformity of the culture result index to the target quality.

In addition, for example, in a case where the target quality information indicates maximization of a yield of a target object per input substrate, and a yield of the target object per input substrate is acquired as a culture result index, one policy trend corresponding to a maximum culture result index (the yield) is selected or a predetermined number of policy trends corresponding to a predetermined number of culture result indexes are selected in descending order of the culture result indexes (the yield). In addition, for example, in a case where the target quality information indicates the minimization of the substrate residual amount, and the substrate residual amount is acquired as a culture result index, one policy trend corresponding to a minimum culture result index (substrate residual amount) is selected or a predetermined number of policy trends corresponding to a predetermined number of culture result indexes are selected in ascending order of the culture result indexes (substrate residual amount).

In the step (S47), the CPU 11 determines the end condition of the optimization of the policy trend. When the end condition is satisfied (S47; YES), the CPU 11 ends the processing, and when the end condition is not satisfied (S47; NO), the policy generation step (S41) and subsequent steps are executed again.

The end condition determined in the step (S47) may be any condition as long as a policy trend most suitable for the target quality can be finally selected, and its concrete condition is not limited. For example, in a mode in which a genetic algorithm is used in the policy generation step (S41), the number of generations of the algorithm is set as the end condition. In addition, the culture result index of the policy trend selected in the previous policy selection step (S45) may be compared with the culture result index of the policy trend selected in the current policy selection step (S45), and the end condition may be set that the current culture result index may have a lower degree of conformity to the target quality than the previous culture result index.

Thus, according to the second support method, it is possible to acquire an optimum policy trend conforming to the target quality, and also acquire an optimum value of the specific culture condition at each culture time point along the optimum policy trend.

This enables automatic design of the control of the fermentative production process by data-driven fermentation simulation. As a result, in the technical development of the fermentative production, it is possible to shift from the conventional know-how type development that has been performed depending on the experience of the developer to the data-driven development, and it is possible to realize the improvement in efficiency and the increase in speed of the development of the fermentative production technology.

Some or all of the above-described embodiments and modifications can also be specified as follows. However, the above-described embodiments and modifications are not limited to the following description.

<1> A fermentative production process support method for supporting a fermentative production process of generating a target object in a culture vessel in which a microorganism is cultured, in which
one or more processors capable of using a learned model that inputs culture information at least including a specific culture condition and outputs a plurality of types of culture state indexes executes:

a culture behavior prediction step of repeating, for each culture time point within a predetermined culture time, a prediction cycle including a first prediction step of acquiring the plurality of types of culture state indexes by inputting the culture information at a certain culture time point to the learned model and a second prediction step of acquiring the culture information at a next culture time point;
a trend acquisition step of acquiring control policy trend information that is a set of indexes to be optimized for each culture time point and in which at least one of the plurality of types of culture state indexes is individually specified as an index to be optimized for each culture time point; and
an optimal condition determination step of determining an optimum value of the specific culture condition correspondingly at each culture time point so that a culture state index specified as an index to be optimized at each culture time point in the acquired control policy trend information among the plurality of types of culture state indexes acquired in the first prediction step is optimized.

<2> The fermentative production process support method according to <1>, in which
the one or more processors further executes:

a target acquisition step of acquiring target quality information;
a policy generation step of generating a plurality of pieces of the control policy trend information;
a result acquisition step of acquiring culture result index corresponding to the target quality information over the predetermined culture time by executing the culture behavior prediction step using the optimum value of the specific culture condition at each culture time point determined in the optimal condition determination step with respect to each of the generated pieces of control policy trend information; and
a policy selection step of selecting one or more pieces of the control policy trend information conforming to the target quality information among the plurality of pieces of control policy trend information on the basis of the culture result index acquired with respect to each of the pieces of control policy trend information.

<3> The fermentative production process support method according to <2>, in which,
in the policy generation step, the one or more processors generate the plurality of pieces of control policy trend information using a genetic algorithm on the basis of the one or more pieces of the control policy trend information selected in the policy selection step.

<4> The fermentative production process support method according to any one of <1> to <3>, in which

in the optimal condition determination step, the one or more processors determine the optimum value of the specific culture condition at each of the culture time points using Bayesian optimization.

<5> The fermentative production process support method according to any one of <1> to <4>, in which

the one or more processors,
in the first prediction step, acquire the plurality of types of culture state indexes including a generation rate of the target object by inputting the culture information including the specific culture condition and a concentration of the target object to the learned model, and
in the second prediction step, calculate a concentration of the target object at the next culture time point at least using a generation rate of the target object included in the acquired plurality of types of culture state indexes.

<6> The fermentative production process support method according to <5>, in which

the specific culture condition includes a temperature in the culture vessel, a hydrogen ion index of a culture solution in the culture vessel, and an oxygen transfer rate or an oxygen consumption rate, and
the one or more processors
in the first prediction step, acquire the plurality of types of culture state indexes including the generation rate of the target object by inputting the culture information including the concentration of the target object, the temperature, the hydrogen ion index, and the oxygen transfer rate or the oxygen consumption rate to the learned model, and
in the second prediction step, acquire the temperature, the hydrogen ion index, and the oxygen transfer rate or the oxygen consumption rate at the next culture time point.

<7> The fermentative production process support method according to any one of <1> to <4>, in which

in the culture vessel, at least the target object, a byproduct, and carbon dioxide are generated and at least a substrate is consumed,
the plurality of types of culture state indexes includes a generation rate of the target object, a generation rate of the byproduct, a generation rate of the carbon dioxide, and a consumption rate of the substrate, and
the index to be optimized for each culture time point indicated by the control policy trend information includes designation of the generation rate of the target object, the generation rate of the byproduct, the generation rate of the carbon dioxide, or the consumption rate of the substrate.

<8> The fermentative production process support method according to any one of <1> to <7>, in which

the learned model outputs only the plurality of types of culture state indexes using only the culture information as an input, and
the culture information and the plurality of types of culture state indexes are configured by an intensive variable or a dimensionless number, or both of them.

<9> A fermentative production process support apparatus including at least a memory and the one or more processors, the fermentative production process support apparatus capable of executing the fermentative production process support method according to any one of <1> to <8>.

Hereinafter, the above-described contents will be described in more detail with reference to examples. However, the description of the following examples does not add any limitation to the contents described above. Examples

In the present example, in the fermentative production process support method according to the second embodiment described above, it has been confirmed that an optimal policy trend can be acquired and an optimum value of the specific culture condition at each culture time point along the acquired policy trend can be acquired, and the practicality of the method is confirmed from the processing time of the method.

A culture experiment was performed with a fermentative production process for producing an organic acid (target object) using aerobic bacteria as a support target. In the culture experiment, various types of culture data were acquired by actually culturing microorganisms (aerobic bacteria) using a predetermined culture tank (aeration stirring tank), and performing sampling at intervals of several hours during the culture experiment. The following learning of the AI model was performed using the acquired culture data. As described later, glucose was used as a substrate.

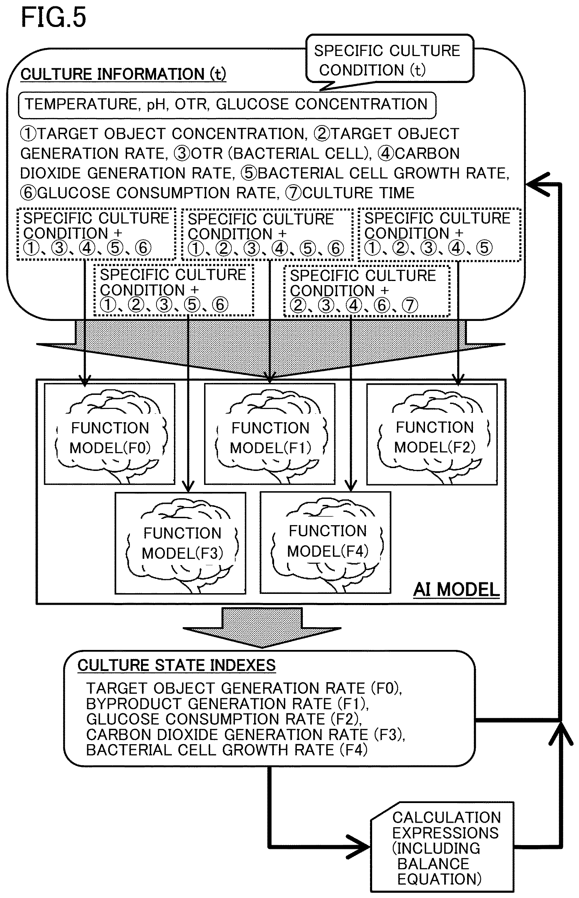

Fig. 5 is a diagram illustrating a data flow of a culture behavior prediction in Examples.

In the present example, an AI model including a combination of five function models F0 to F4 is used. Each of the function models F0 to F4 is a function that approximately expresses the bacterial cell body activity response, and is constructed using regression of supervised learning with the culture information as an input variable and one type of the culture state indexes as an output variable. The input variables and the output variables of each function model are as follows.

<Output Variables>

- Function model F0 = target object generation rate per unit bacterial cell weight
- Function model F1 = byproduct production rate per unit bacterial cell weight
- Function model F2 = glucose consumption rate per unit bacterial cell weight
- Function model F3 = bacterial cell growth rate per unit bacterial cell weight
- Function model F4 = carbon dioxide generation rate per unit bacterial cell weight

<Input Variables>

- Function model F0 = temperature, pH, oxygen transfer rate (OTR), glucose concentration, target object concentration, oxygen transfer rate per unit bacterial cell weight, carbon dioxide generation rate per unit bacterial cell weight, bacterial cell growth rate per unit bacterial cell weight, and glucose consumption rate per unit bacterial cell weight
- Function model F1 = temperature, pH, oxygen transfer rate (OTR), glucose concentration, target object concentration, target object generation rate per unit bacterial cell weight, oxygen transfer rate per unit bacterial cell weight, carbon dioxide generation rate per unit bacterial cell weight, bacterial cell growth rate per unit bacterial cell weight, and glucose consumption rate per unit bacterial cell weight
- Function model F2 = temperature, pH, oxygen transfer rate (OTR), glucose concentration, target object generation rate per unit bacterial cell weight, target object concentration, oxygen transfer rate per unit bacterial cell weight, carbon dioxide generation rate per unit bacterial cell weight, and bacterial cell growth rate per unit bacterial cell weight
- Function model F3 = temperature, pH, oxygen transfer rate (OTR), glucose concentration, target object generation rate per unit bacterial cell weight, target object concentration, oxygen transfer rate per unit bacterial cell weight, bacterial cell growth rate per unit bacterial cell weight, and glucose consumption rate per unit bacterial cell weight
- Function model F4 = temperature, pH, oxygen transfer rate (OTR), glucose concentration, culture time, target object generation rate per unit bacterial cell weight, oxygen transfer rate per unit bacterial cell weight, carbon dioxide generation rate per unit bacterial cell weight, and glucose consumption rate per unit bacterial cell weight

As in the present example, the output variables of the five function models are set as the five types of culture state indexes as the output information of the AI model. In the present example, the control policy trend information (policy trend) had a format in which one type of the five types of culture state indexes was designated as a target index to be optimized for each culture time point. That is, the target object generation rate per unit bacterial cell weight is indicated by the identifier "0", the byproduct production rate per unit bacterial cell weight is indicated by the identifier "1", the glucose consumption rate per unit bacterial cell weight is indicated by the identifier "2", the bacterial cell growth rate per unit bacterial cell weight is indicated by the identifier "3", and the carbon dioxide generation rate per unit bacterial cell weight is indicated by the identifier "4". For example, the policy trend can be generated as follows.

```
Policy trend = [44422103...]
```

In the present example, the culture time was set to 72 hours, and the optimization interval was set to 5 minutes. Therefore, 864 indexes to be optimized are designated every 5 minutes in the policy trend.

In the present example, in each of the five function models, at least part of the culture information as the input information of the AI model was used as an input variable, and the specific culture condition included in the culture information were temperature, pH, oxygen transfer rate (OTR), and glucose concentration.

Then, in the optimal condition determination step (S32), the optimum value of such a specific culture condition was determined. In the present example, Bayesian optimization was adopted for optimization of the specific culture condition.

As in the present example, in the first prediction step (S33) of the culture behavior prediction step, when culture information at a certain culture time point was input to the input variable of each of the five function models, the above-described five types of culture state indexes (rate parameters) were acquired as the values of the output variables of the five function models.

In the second prediction step (S34) of the culture behavior prediction step, culture information at the next culture time point was acquired on the basis of the five types of culture state indexes acquired in the first prediction step (S33). The target object generation rate obtained from the function model F0, the glucose consumption rate obtained from the function model F2, the carbon dioxide generation rate obtained from the function model F3, and the bacterial cell growth rate obtained from the function model F4 were each used as culture information at the next culture time point. The other culture information at the next culture time point was derived using the substance balance equations as in the above equations (1) to (5), other calculation expressions, the five types of culture state indexes acquired in the first prediction step (S33), and the like.

In the present example, the target quality information was set to "maximization of the target object production amount at the culture end time" (target acquisition step (S40)), and a genetic algorithm was adopted to optimize the policy trend (50 individuals, 50 generation).

In the present example, a computer having the following specifications was caused to execute the fermentative production process support method.

- CPU = Intel $^®$ Xeon $^®$ Gold 6258R 28 Core 56 threads x 2
- OS = Windows10 Pro
- RAM = 128GB

As a result, optimal control policy trend information and an optimum value of the specific culture condition at each culture time point along the control policy trend information were acquired by the processing time of about 18.6 hours.

Furthermore, in the present example, using the thus acquired optimal control policy trend information, and the optimum value of the specific culture condition at each culture time point in accordance with the control policy trend information, a demonstration experiment was conducted under the same condition (aerobic bacterial species, culture tank scale, and the like) as those in the culture experiment described above.

In this demonstration experiment, cultivation was actually performed for 72 hours, and the production amount (g) of the target object was measured at timings after the lapse of 7 hours, 24 hours, 32 hours, 48 hours, and 72 hours. On the other hand, a prediction value (g) of the target object production amount at each of the timings was derived in the culture behavior prediction step of the present example, and a measurement value and the prediction value of the target object production amount at each timing were normalized using the prediction value of the target object production amount after the lapse of 72 hours (g/g-MAX).

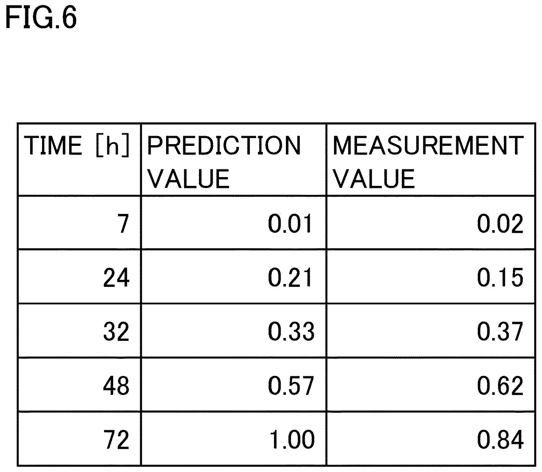

Fig. 6 is a table showing results of the demonstration experiment.

In Fig. 6, the culture elapsed time is indicated in the column of time, and the prediction value and the measurement value of the target object production amount normalized as described above are indicated in the respective columns of the prediction value and the measurement value.

As a result, the prediction error of the target object production amount after the lapse of 72 hours was suppressed to 16%, and the predicted production trend of the target object roughly matched the experimental result.

Furthermore, in the culture according to the optimum value of the specific culture condition obtained in the present example, a result was obtained in which the final production amount of the target object was improved by 50 as compared with the conventional value before examination of the example.

According to the present example, in the fermentative production process support method according to the second embodiment, it has been thus confirmed that an optimal policy trend can be acquired and an optimum value of the specific culture condition at each culture time point along the acquired policy trend can be acquired, and the practicality of the method has been confirmed from viewpoint of the processing time.

This application claims priority based on Japanese Patent Application No. 2022-194671 filed on December 6, 2022, which is entirely incorporated herein by reference.

REFERENCE SIGNS LIST

10 Information processing apparatus (fermentative production process support apparatus)
11 CPU
12 Memory
13 Input and output I/F
14 Communication unit

15    Display device
16    Input device

**Claims**

1.  A fermentative production process support method for supporting a fermentative production process of generating a target object in a culture vessel in which a microorganism is cultured, wherein
    one or more processors capable of using a learned model that inputs culture information at least including a specific culture condition and outputs a plurality of types of culture state indexes executes:

    a culture behavior prediction step of repeating, for each culture time point within a predetermined culture time, a prediction cycle including a first prediction step of acquiring the plurality of types of culture state indexes by inputting the culture information at a certain culture time point to the learned model and a second prediction step of acquiring the culture information at a next culture time point;
    a trend acquisition step of acquiring control policy trend information that is a set of indexes to be optimized for each culture time point and in which at least one of the plurality of types of culture state indexes is individually specified as an index to be optimized for each culture time point; and
    an optimal condition determination step of determining an optimum value of the specific culture condition correspondingly at each culture time point so that a culture state index specified as an index to be optimized at each culture time point in the acquired control policy trend information among the plurality of types of culture state indexes acquired in the first prediction step is optimized.

2.  The fermentative production process support method according to claim 1, wherein
    the one or more processors further executes:

    a target acquisition step of acquiring target quality information;
    a policy generation step of generating a plurality of pieces of the control policy trend information;
    a result acquisition step of acquiring culture result index corresponding to the target quality information over the predetermined culture time by executing the culture behavior prediction step using the optimum value of the specific culture condition at each culture time point determined in the optimal condition determination step with respect to each of the generated pieces of control policy trend information; and
    a policy selection step of selecting one or more pieces of the control policy trend information conforming to the target quality information among the plurality of pieces of control policy trend information on a basis of the culture result index acquired with respect to each of the pieces of control policy trend information.

3.  The fermentative production process support method according to claim 2, wherein,
    in the policy generation step, the one or more processors generate the plurality of pieces of control policy trend information using a genetic algorithm on the basis of the one or more pieces of the control policy trend information selected in the policy selection step.

4.  The fermentative production process support method according to any one of claims 1 to 3, wherein
    in the optimal condition determination step, the one or more processors determine the optimum value of the specific culture condition at each of the culture time points using Bayesian optimization.

5.  The fermentative production process support method according to any one of claims 1 to 4, wherein

    the one or more processors,
    in the first prediction step, acquire the plurality of types of culture state indexes including a generation rate of the target object by inputting the culture information including the specific culture condition and a concentration of the target object to the learned model, and
    in the second prediction step, calculate a concentration of the target object at the next culture time point at least using a generation rate of the target object included in the acquired plurality of types of culture state indexes.

6.  The fermentative production process support method according to claim 5, wherein

    the specific culture condition includes a temperature in the culture vessel, a hydrogen ion index of a culture solution in the culture vessel, and an oxygen transfer rate or an oxygen consumption rate, and
    the one or more processors

in the first prediction step, acquire the plurality of types of culture state indexes including the generation rate of the target object by inputting the culture information including the concentration of the target object, the temperature, the hydrogen ion index, and the oxygen transfer rate or the oxygen consumption rate to the learned model, and in the second prediction step, acquire the temperature, the hydrogen ion index, and the oxygen transfer rate or the oxygen consumption rate at the next culture time point.

7. The fermentative production process support method according to any one of claims 1 to 4, wherein

in the culture vessel, at least the target object, a byproduct, and carbon dioxide are generated and at least a substrate is consumed,
the plurality of types of culture state indexes includes a generation rate of the target object, a generation rate of the byproduct, a generation rate of the carbon dioxide, and a consumption rate of the substrate, and
the index to be optimized for each culture time point indicated by the control policy trend information includes designation of the generation rate of the target object, the generation rate of the byproduct, the generation rate of the carbon dioxide, or the consumption rate of the substrate.

8. The fermentative production process support method according to any one of claims 1 to 7, wherein

the learned model outputs only the plurality of types of culture state indexes using only the culture information as an input, and
the culture information and the plurality of types of culture state indexes are configured by an intensive variable or a dimensionless number, or both of them.

9. A fermentative production process support apparatus comprising at least a memory and the one or more processors, the fermentative production process support apparatus capable of executing the fermentative production process support method according to any one of claims 1 to 8.

# FIG.1

MICROORGANISM
SPECIFIC
BACTERIAL CELL

TARGET
PRODUCT

BYPRODUCT

CULTURE MEDIUM
COMPONENT
(SUBSTRATE)

SUBSTRATE
INPUT
SOLUTION

CULTURE
SOLUTION

TEMPERATURE

pH

AIR (OXYGEN)

FIG.2

# FIG.3

```
           ┌──────────────┐
           │    START     │
           └──────┬───────┘
                  │
                  ▼
        ┌───────────────────────┐
        │ TREND ACQUISITION STEP │ ～ S31
        └───────────┬───────────┘
                    │
                    ▼
        ┌───────────────────────────────┐
        │      INDEX TO BE OPTIMIZED     │ ～ L1(S)
        │  (FOR EACH CULTURE TIME POINT t) │
        └───────────────┬───────────────┘
                        │
                        ▼
   ┌─────────────────────────────────────┐
   │       OPTIMAL CONDITION             │ ～ S32
   │       DETERMINATION STEP            │
   │  ┌──────────────────────────────┐   │
   │  │     CULTURE BEHAVIOR         │   │ ～ S33
   │  │     PREDICTION STEP          │   │
   │  │   (FIRST PREDICTION STEP)    │   │
   │  └──────────────────────────────┘   │
   └─────────────────┬───────────────────┘
                     │
                     ▼
   ┌─────────────────────────────────────┐
   │       CULTURE BEHAVIOR              │ ～ S34
   │       PREDICTION STEP               │
   │   (SECOND PREDICTION STEP)          │
   └─────────────────┬───────────────────┘
                     │
                     ▼
   ┌─────────────────────────────────────┐
   │       INDEX TO BE OPTIMIZED         │ ～ L1(E)
   │  (PREDETERMINED CULTURE TIME)       │
   └─────────────────┬───────────────────┘
                     │
                     ▼
           ┌──────────────┐
           │     END      │
           └──────────────┘
```

# FIG.4

```
        START
          │
          ▼
┌─────────────────────────┐
│ TARGET ACQUISITION STEP │  S40
└─────────────────────────┘
          │
          ▼
┌─────────────────────────┐
│ POLICY GENERATION STEP  │  S41
└─────────────────────────┘
          │
          ▼
┌─────────────────────────┐
│ CONTROL POLICY TREND    │  L2(S)
│ INFORMATION (INDIVIDUAL)│
└─────────────────────────┘
          │
          ▼
      ┌─────────┐
      │ FIG.3   │
      │ FLOW    │
      └─────────┘
          │
          ▼
┌─────────────────────────┐
│ RESULT ACQUISITION STEP │  S43
└─────────────────────────┘
          │
          ▼
┌─────────────────────────┐
│ CONTROL POLICY TREND    │  L2(E)
│ INFORMATION (ALL)       │
└─────────────────────────┘
          │
          ▼
┌─────────────────────────┐
│ POLICY SELECTION STEP   │  S45
└─────────────────────────┘
          │
          ▼
        END ?   S47
     NO  │  YES
          ▼
         END
```

# FIG.5

SPECIFIC CULTURE CONDITION (t)

**CULTURE INFORMATION (t)**

TEMPERATURE, pH, OTR, GLUCOSE CONCENTRATION

①TARGET OBJECT CONCENTRATION, ②TARGET OBJECT GENERATION RATE, ③OTR (BACTERIAL CELL), ④CARBON DIOXIDE GENERATION RATE, ⑤BACTERIAL CELL GROWTH RATE, ⑥GLUCOSE CONSUMPTION RATE, ⑦CULTURE TIME

SPECIFIC CULTURE CONDITION + ①、③、④、⑤、⑥

SPECIFIC CULTURE CONDITION + ①、②、③、④、⑤、⑥

SPECIFIC CULTURE CONDITION + ①、②、③、④、⑤

SPECIFIC CULTURE CONDITION + ①、②、③、⑤、⑥

SPECIFIC CULTURE CONDITION + ②、③、④、⑥、⑦

FUNCTION MODEL(F0)

FUNCTION MODEL(F1)

FUNCTION MODEL(F2)

FUNCTION MODEL(F3)

FUNCTION MODEL(F4)

**AI MODEL**

**CULTURE STATE INDEXES**

TARGET OBJECT GENERATION RATE (F0), BYPRODUCT GENERATION RATE (F1), GLUCOSE CONSUMPTION RATE (F2), CARBON DIOXIDE GENERATION RATE (F3), BACTERIAL CELL GROWTH RATE (F4)

CALCULATION EXPRESSIONS (INCLUDING BALANCE EQUATION)

FIG.6

| TIME [h] | PREDICTION VALUE | MEASUREMENT VALUE |
|---|---|---|
| 7 | 0.01 | 0.02 |
| 24 | 0.21 | 0.15 |
| 32 | 0.33 | 0.37 |
| 48 | 0.57 | 0.62 |
| 72 | 1.00 | 0.84 |

## INTERNATIONAL SEARCH REPORT

| | International application No. |
|---|---|
| | **PCT/JP2023/043574** |

**A. CLASSIFICATION OF SUBJECT MATTER**

*C12N 1/00*(2006.01)i; *C12M 1/00*(2006.01)i
FI: C12N1/00 B; C12M1/00 C

According to International Patent Classification (IPC) or to both national classification and IPC

**B. FIELDS SEARCHED**

Minimum documentation searched (classification system followed by classification symbols)

C12N1/00; C12M1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2020/205611 A1 (AMGEN INC.) 08 October 2020 (2020-10-08) paragraphs [0001]-[0007], [0046], [00116] | 1-9 |
| A | WO 2019/163304 A1 (NIKON CORPORATION) 29 August 2019 (2019-08-29) claims 1-2, paragraphs [0089]-[0093] | 1-9 |
| A | WALSH, Ian et al., Harnessing the potential of machine learning for advancing "Quality by Design" in biomanufacturing, Mabs, 09 January 2022, vol. 14, no. 1, e2013593, pages 1-11, https://doi.org/10.1080/19420862.2021.2013593 particularly, abstract, figures | 1-9 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **13 February 2024** | **20 February 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2023/043574**

| Patent document cited in search report | | | Publication date (day/month/year) | Patent family member(s) | Publication date (day/month/year) |
|---|---|---|---|---|---|
| WO | 2020/205611 | A1 | 08 October 2020 | US 2022/0208297 A1 paragraphs [0001]-[0007], [0046], [0116] | |
| WO | 2019/163304 | A1 | 29 August 2019 | JP 2022-185038 A claim 1, paragraphs [0087]-[0091] | |

Form PCT/ISA/210 (patent family annex) (July 2022)

**REFERENCES CITED IN THE DESCRIPTION**

**Patent documents cited in the description**

- WO 2021166824 A
- JP 2022194671 A